# EUROPÄISCHE PATENTSCHRIFT

(11) **EP 1 380 839 B1**
(45) Veröffentlichungstag und Bekanntmachung des Hinweises auf die Patenterteilung: **31.03.2010**
(21) Anmeldenummer: 03019025.0
(22) Anmeldetag: 17.02.1999
(51) Int. Cl.: G01N 33/543

(54) **Erzeugung räumlich scharf begrenzter Festphasen für Bindungsassays**
Production of spatially defined solid phases for binding assays
Production de phases solides spatialement définies pour l'essai à liaison

(30) Priorität: 19.02.1998 DE 19806989
(43) Veröffentlichungstag der Anmeldung: 14.01.2004
(62) Teilanmeldung aus: 99103135.2
(73) Patentinhaber: Roche Diagnostics GmbH, 68305 Mannheim (DE)
(72) Erfinder: Hornauer, Hans, Dr., 82380 Peissenberg (DE)
(74) Vertreter: Dey, Michael

(56) Entgegenhaltungen:
- EP-A- 0 427 984
- US-A- 4 591 570
- PIRRUNG MICHAEL C ET AL: "A general method for the spatially defined immobilization of biomolecules on glass surfaces using "caged" biotin." BIOCONJUGATE CHEMISTRY, Bd. 7, Nr. 3, 1996, Seiten 317-321, XP002095758 ISSN: 1043-1802
- DELAMARCHE E ET AL: "Microfluidic networks for chemical patterning of substrates: Design and application to bioassays" JOURNAL OF THE AMERICAN CHEMICAL SOCIETY 28 JAN 1998 UNITED STATES, Bd. 120, Nr. 3, 28. Januar 1998 (1998-01-28), Seiten 500-508, XP002957278 ISSN: 0002-7863
- SUNDARABABU G ET AL: "PHOTOCHEMICAL LINKAGE OF ANTIBODIES TO SILICON CHIPS" PHOTOCHEMISTRY AND PHOTOBIOLOGY, OXFORD, GB, Bd. 61, Nr. 6, 1995, Seiten 540-544, XP001077098 ISSN: 0031-8655
- ANSARI A A ET AL: "ELISA SOLID PHASE STABILITY AND BINDING CHARACTERISTICS" JOURNAL OF IMMUNOLOGICAL METHODS, Bd. 84, Nr. 1-2, 1985, Seiten 117-124, XP009020234 ISSN: 0022-1759

## Beschreibung

Die Erfindung betrifft ein Verfahren zum Aufbringen einer mehrlagigen Beschichtung auf einen festen, nicht porösen Träger, ein Verfahren zur Vermeidung des Verschmierens von räumlich begrenzten Festphasen, ein Verfahren zur Verringerung der unspezifischen Bindung an eine mit Streptavidin oder Avidin beschichtete Festphase sowie Festphasen, die mit dem erfindungsgemäßen Verfahren hergestellt werden.

Bindungsassays zur Bestimmung von Analyten, wie etwa Immunoassays, Nukleinsäurehybridisierungsassays usw. werden in großem Umfang eingesetzt. Der Nachweis eines Analyten durch Bindungsassays kann mit einer heterogenen Testführung erfolgen, wobei eine flüssige Phase mit einer Festphase in Kontakt gebracht wird. Die Festphase umfaßt Testflächen mit Rezeptormolekülen, an die der Analyt spezifisch bindefähig ist. Die Bindung des Analyten wird anschließend, z.B. mittels einer Markierung, nachgewiesen.

Für eine zuverlässige qualitative oder quantitative Bestimmung eines Analyten, ist es notwendig, die Testflächen der Bindungsassays reproduzierbar und mit genau definierten Mengen an Rezeptormolekülen herstellen zu können. Unterschiede in der Größe der Testflächen, Randunschärfe der Testflächen, Verschmieren der Rezeptormoleküle, unterschiedliche Rezeptordichte in den Testflächen und unspezifische Bindestellen führen zu Unterschieden in der detektierten Signalhöhe und somit zu Ungenauigkeiten bei der Auswertung von Testergebnissen.

Diese Probleme wirken sich zumeist umso stärker aus, je kleiner die Festphase und die darauf befindlichen Testflächen sind. Liegen einzelne Testflächen mit unterschiedlichen Rezeptormolekülen zum Nachweis verschiedener Analyten nahe beieinander, so kann es durch Randunschärfe der Testflächen oder/und Verschmieren der Rezeptormoleküle zur Kontamination spezifischer Testflächen mit gegen einen anderen Analyten gerichteten Rezeptormolekülen kommen, was zu falschen Testergebnissen führen kann. Besonders schwerwiegend ist dieses Problem bei qualitativen Tests zum Nachweis von Infektionskrankheiten. So hat z.B. eine aufgrund von Verschmieren der Rezeptormoleküle falsch positive Probe bei einem HIV-Test erhebliche Konsequenzen.

Eine Vielzahl von Testformaten beruht auf der Verwendung von porösen Trägermaterialien wie etwa Papierscheiben oder anderen porösen Materialien. Bei solchen Testformaten, wie sie beispielsweise in der EP-A-0 427 984 und der EP-A-0 063 810 beschrieben sind, wird eine Rezeptorschicht auf einem porösen Material gebildet, indem Rezeptormoleküle aus einer Lösung auf das poröse Material aufgebracht und durch das Trägermaterial aufgesaugt wurden. Das Aufsaugen der Beschichtungslösung bewirkt insbesondere eine von der lokalen Mikrostruktur des Trägermaterials abhängige Aufweitung der beschichteten Fläche. Es ist deshalb schwierig, mit dieser Methode reproduzierbare gleichmäßige Testflächen, insbesondere in miniaturisierten Testformaten bereitzustellen. Zudem ist bei solchen miniaturisierten Testformaten auf Basis von porösen Trägermaterialien die Auswertung schwierig. Durch die Unebenheit und Ungleichmäßigkeit der Oberfläche poröser Materialien kann zumeist nur eine begrenzte optische Auflösung der Testflächen erreicht werden.

Es wurde deshalb versucht, anstelle von porösem Trägermaterial ein Trägermaterial mit einer nichtporösen Oberfläche zu verwenden. US-PS-4,591,570 beschreibt eine Immunoassay-Vorrichtung zur gleichzeitigen Bestimmung mehrerer Antigene mit Hilfe eines Antikörperarrays, wobei als Träger Glas oder Kunststoff verwendet wird. Durch die Verwendung dieses Trägermaterials ist es möglich, die Gesamtgröße des Testformats und somit die benötigte Probenmenge deutlich zu verringern. Allerdings tritt auch bei nichtporösen Oberflächen, insbesondere bei miniaturisierten Testverfahren, das Problem auf, dass die Rezeptormoleküle verschmieren. Ein Verschmieren kann durch unkontrolliertes Spriten der aufgebrachten Beschichtungslösung, d. h. eine Flächenzunahme, bei der die Kontur regelmäßig bleibt, verursacht werden. Daneben kann auch ein unkontrolliertes Zerfliessen der aufgebrachten Beschichtungslösung auftreten, was zu einer unregelmäßigen Kontur führt. Schließlich kann das Verschmieren eine Verfrachtung von Rezeptormolekülen in den Bereich ausserhalb der mit Beschichtungslösung benetzten Flächen bewirken.

Weiterhin beschreibt Pirrung et al., Bioconjugate Chemistry, 7(3), 1996, 317 - 321 ein Verfahren, bei dem BSA mit einer durch Licht entfernbaren Nitrobenzylgruppe verbunden wird. Nachdem BSA an einen Glasträger gebunden wurde, kann mittels Bestrahlung BSA an bestimmten Stellen entfernt werden, die dann für eine Avidinbindung zur Verfügung stehen. Anschließende Zugabe eines biotinylierten Moleküls erlaubt eine ortsspezifische Bindung des Moleküls.

Delamarche et al., J. Am. Soc., 120, 1998, 500-508 beschreibt die Anordnung, Funktion und Bindung einer räumlich begrenzten Anordnung chemischer Reaktanten auf einem Substrat mittels eines Mikroflüssigkeitsnetzwerks. Mikroflüssigkeitsnetzwerke arbeiten im allgemeinen mit Flüssigkeitsmengen im µl-Bereich und eignen sich daher für Anwendungen, bei denen geringe Mengen an Reaktanten zur Verfügung stehen.

Sundarababu et al., Photochemistry and Photobiology 61(6), 1995, 540-544 beschreibt die Beschichtung von Siliziumchips mit Antikörpern mittels einer lichtinduzierten Kopplung.

Ansari et al., Journal of Immunological Methods, 84, 1985, 117-124 beschreibt die Beschichtung von Polystyrolmikrotiterplatten mit Antikörpern und untersucht die Auswirkung unterschiedlicher Lagerbedingungen der Platten auf die Stabilität und Bindung der Antikörper.

Viele der üblicherweise verwendeten Rezeptormoleküle, wie etwa niedermolekulare Rezeptormoleküle, adsorbieren zudem nur schlecht oder schlecht reproduzierbar auf festen Trägern mit nichtporösen Oberflächen. Weiterhin enthalten viele wässrige Lösungen von Rezeptormolekülen, wie sie üblicherweise zum Aufbringen von Rezeptormolekülen auf Festphasen verwendet werden, zum Stabilisieren ein Detergens, wodurch die Adsorption der Rezeptormoleküle auf feste Träger mit nichtporösen Oberflächen zumindest zum Teil verhindert wird.

Um die Bindefähigkeit von Rezeptormolekülen an nichtporöse Oberflächen zu verbessern, können mehrlagige Beschichtungen aufgebracht werden. Aber auch bei solchen Beschichtungen treten innerhalb einzelner Testflächen Unterschiede in der Größe und Randunschärfen auf, was bei der Auswertung, insbesondere von miniaturisierten Testformaten, erhebliche Probleme ergibt.

Eine Aufgabe der vorliegenden Erfindung war es deshalb, ein Verfahren bereitzustellen, mit dem räumlich scharf begrenzte Testflächen für Bindungsassays erhalten werden können.

Diese Aufgabe wird erfindungsgemäß gelöst durch ein Verfahren zum Aufbringen von Rezeptormolekülen enthaltenden räumlich begrenzten Flächen auf einen festen, nicht porösen Träger, umfassend die Schritte:
(a) Aufbringen einer Rezeptormoleküle enthaltenden Beschichtungslösung auf vorbestimmte begrenzte Bereiche des festen Trägers,
(b) Binden der Rezeptormoleküle in den räumlich begrenzten Flächen,
(c) Trocknen der Beschichtungslösung und
(d) Aufbringen einer Nachbeladelösung, wobei zu der Beschichtungslösung ein Eluationsverzögerer zugegeben wird, wobei der Elutionsverzögerer ausgewählt ist aus Saccariden, Polyvinylpyrrolidon, Dextran, Gelantine oder Zellulosederivaten, wobei die Nachbeladelösung eine Blockierungssubstanz enthält. Weiterhin kann das erfindungsgemäße Verfahren die eine Vorbeschichtung umfassenden Schritte:
   (a') Aufbringen einer Vorbeschichtung auf ein Reagenzfeld des festen Trägers,
   (b') Waschen des vorbeschichteten Trägers mit einer wässrigen Flüssigkeit und
   (c') Aufbringen einer zweiten Beschichtung, umfassend Rezeptormoleküle, die mit der Vorbeschichtung bindefähig sind, auf den vorbeschichteten Träger in Form von räumlich begrenzten Flächen auf dem Reagenzfeld,
   enthalten.

Erfindungsgemäß wird der feste, nichtporöse Träger zunächst mit einer Vorbeschichtung versehen. Die Vorbeschichtung kann dabei vollflächig auf einen Teil oder auf die gesamte Fläche eines Reagenzfeldes des festen Trägers oder auch in Form von Spots aufgetragen werden. Bevorzugt wird die Vorbeschichtung vollflächig aufgetragen. Die Vorbeschichtung wird typischerweise aus einer wässrigen Lösung auf den Träger aufgebracht. Sie kann aus beliebigen Molekülen bestehen, welche die Bindung einer zweiten Beschichtung ermöglichen. Bevorzugt umfasst die Vorbeschichtung einen ersten Partner eines hochaffinen Bindepaares, wie z. B. Streptavidin, Avidin oder Biotin sowie Analoga, Derivate und Konjugate der vorstehenden Substanzen, oder Antikörper wie z. B. Anti-Maus-Antikörper. Es können aber auch Moleküle als Vorbeschichtung aufgebracht werden, die für eine kovalente Bindung mit der zweiten Beschichtung vorgesehen sind, etwa Moleküle, die eine Amin-, eine Sulfid- oder eine Silylgruppe enthalten.

Wenn man auf eine solche Vorbeschichtung Rezeptormoleküle in wässriger Lösung in Form kleiner Tröpfchen aufbringt, diffundieren sie aus der Lösung an die Vorbeschichtung und binden an diese. Es wurde jedoch festgestellt, dass die aufgebrachten Flüssigkeitströpfchen verlaufen, was zu unterschiedlicher Kontur und Größe der Spotflächen führt. Daraus resultieren

Unterschiede in der Rezeptordichte in den einzelnen Testflächen und somit Unterschiede in der Signalhöhe die zu falschen Resultaten führen können.

Überraschenderweise wurde nun festgestellt, dass durch Einführen des Verfahrenschrittes (b'), nämlich Waschen des vorbeschichteten Trägers mit einer wässrigen Flüssigkeit, reproduzierbare, gleichförmige Testspots erhalten werden können. Das Waschen des vorbeschichteten Trägers kann mit reinem Wasser durchgeführt werden, bevorzugt wird ein Puffer mit geringer Ionenstärke verwendet. Die wässrige Flüssigkeit oder Waschlösung, die zum Waschen des vorbeschichteten Trägers verwendet wird, kann Puffersubstanzen, Detergenzien oder/und sonstige Zusätze umfassen. Als Puffersubstanzen können grundsätzlich alle gebräuchlichen oder dem Fachmann bekannten Puffersubstanzen verwendet werden. Die Konzentration, in der die Puffersubstanzen in der Waschlösung vorliegen beträgt bevorzugt ≤ 250 mM, besonders bevorzugt ≤ 100 mM und am meisten bevorzugt ≤ 40 mM. Die besten Ergebnisse im Hinblick auf die erfindungsgemäße Funktion als Waschlösung werden dann erhalten, wenn die Puffersubstanz in einer Konzentration ≤ 10 mM, bevorzugt ≤ 5 5 mM und am meisten bevorzugt ≤ 2 mM vorliegt. In Tabelle 1 sind einige Puffersubstanzen, sowie ihre bevorzugten (c₁), besonders bevorzugten (c₂) und am meisten bevorzugten (c₃) Konzentrationsbereiche angegeben.

**Tabelle 1**

| **Puffersubstanz** | **c₁ [mM]** | **c₂ [mM]** | **c₃ [mM]** |
|---|---|---|---|
| Phosphat | ≤ 40 | ≤ 5 | ≤ 2 |
| Carbonat | ≤ 40 | ≤ 5 | ≤ 2 |
| Mes/tris | ≤ 100 | ≤ 10 | ≤ 5 |
| Hepes/Tries | ≤ 100 | ≤ 10 | ≤ 5 |

Für eine weitere Optimierung des erfindungsgemäßen Verfahrens ist es möglich, in Abhängigkeit von der Art der Wechselwirkung, mit der die Rezeptormoleküle auf der Vorbeschichtung gebunden werden, einen Puffer auszuwählen, der optimale Reaktionsbedingungen gewährleistet. Beispielsweise ist für eine Vorbeschichtung mit Streptavidin der pH-Bereich von 5 bis 9 optimal.

Erfindungsgemäß können auch Pufferkombinationen verwendet werden, die eine flüchtige Komponente enthalten, wie etwa Essigsäure oder Ameisensäure. Beim Eintrocknen von Pufferresten können solche Pufferkombinationen jedoch auf der Vorbeschichtung zu großen pH-Schwankungen führen, da lediglich ein Bestandteil des Puffers verdampft, während der andere auf der Oberfläche verbleibt. Bevorzugt werden deshalb Pufferkombinationen verwendet, die ausschließlich aus flüchtigen oder ausschließlich aus nichtflüchtigen Komponenten bestehen.

Zusätzlich oder anstelle von Puffersubstanzen kann die erfindungsgemäß verwendete Waschlösung auch ein Detergenz enthalten. Ein Zusatz von Detergenz zur Waschlösung unterstützt das weitgehend rückstandsfreie Entfernen der Waschlösung von der Vorbeschichtung. Das Detergenz ist in der Waschlösung bevorzugt in einer Konzentration ≤ 0,05 Vol.-% (bezogen auf das Gesamtvolumen), insbesondere ≤ 0,02 % (vol-vol), mehr bevorzugt ≤ 0,01 % (vol-vol) und am meisten bevorzugt ≤ 0,005 % (vol-vol) enthalten. Die besten Ergebnisse werden erhalten, wenn die Konzentration des Detergenz in der Waschlösung ≤ 0,003 % (vol-vol), mehr bevorzugt ≤ 0,002 % (vol-vol) und am meisten bevorzugt ≤ 0,001 % (vol-vol) beträgt. Die bevorzugten (c₁), besonders bevorzugten (c₂) und am meisten bevorzugten (c₃) Konzentrationen für einige bevorzugte Detergenzien sind in Tabelle 2 angegeben.

**Tabelle 2**

| **Detergenz** | **c₁ [% vol-vol)]** | **c₂ [% (vol-vol)]** | **c₃ [% (vol-vol)]** |
|---|---|---|---|
| Tween 20 | ≤ 0,01 | ≤ 0,005 | ≤ 0,003 |
| n-Octylglucosid | ≤ 0,02 | ≤ 0,01 | ≤ 0,003 |
| Chaps | ≤ 0,01 | ≤ 0,005 | ≤ 0,003 |
| Brj 35 | ≤ 0,005 | ≤ 0,002 | ≤ 0,001 |

Grundsätzlich sind alle Detergenzien geeignet, ein weitgehend rückstandsfreies Entfernen der Waschlösung zu erzielen. Die Eignung eines bestimmten Detergenz für ein bestimmtes Testformat kann abhängig vom Typ der Beschichtung und der technischen Randbedingungen, wie etwa dem Format des Reagenzträgers, der verwendeten Absaugvorrichtung usw. vom Fachmann durch wenige Vorversuche ermittelt werden. Insbesondere muß darauf geachtet werden, daß das Detergenz eine später aufgebrachte Flüssigkeit nicht oder nur geringfügig zum Zerfließen bringt. Aus diesem Grund ist beispielsweise bei der Verwendung des Detergenz SDS besondere Aufmerksamkeit, insbesondere hinsichtlich der verwendeten Konzentration angebracht.

Die erfindungsgemäß verwendete Waschlösung kann auch sonstige Zusätze umfassen, die sich vorteilhaft auf die Verfahrensführung auswirken. Insbesondere können Zusätze verwendet werden, die stabilisierend auf die Vorbeschichtung wirken. Dies ermöglicht eine zeitliche Trennung zwischen der Vorbeschichtung des Reagenzträgers und der Aufbringung der für den jeweiligen Bindeassay spezifischen Beschichtung im Herstellungsverfahren. Beispiele für solche sonstige Zusätze sind Zucker, Salze und Blockreagenzien. Die bevorzugten (c₁), besonders bevorzugten (c₂) und am meisten bevorzugten (c₃) Konzentrationen für diese Substanzen sind in Tabelle 3 dargestellt.

**Tabelle 3**

| **Substanz** | **c₁ [% (Gew.-Vol)]** | **c₂ [% (Gew.-Vol)]** | **c₃ [% (Gew.-Vol)]** |
|---|---|---|---|
| Zucker | ≤ 0,5 | ≤ 0,1 | ≤ 0,05 |
| Salze | ≤ 0,5 | ≤ 0,1 | ≤ 0,05 |
| Blockreagenzien | ≤ 0.2 | ≤ 0,02 | ≤ 0,005 |

Bevorzugte Zucker sind niedermolekulare Zucker, wie etwa Saccharose, mit einem Molekulargewicht ≤1000, besonders bevorzugt ≤ 500. Am meisten bevorzugt sind niedermolekulare Zuckeroligomere, die eine, zwei oder drei Zuckereinheiten umfassen.

Als Salze werden bevorzugt solche verwendet, die während des Eintrocknens nicht zur Kristallbildung neigen, wie etwa Di-Natriumtatrat-dihydrat.

Als Blockreagenzien werden bevorzugt niedermolekulare Proteine, wie etwa Pepton verwendet.

Die Konzentration der einzelnen Stoffe in der Waschlösung kann in Abhängigkeit von den spezifischen Testanforderungen, wie etwa Art der Beschichtung, Größe des Testformats usw. eingestellt werden. so wird beispielsweise im Fall von Reagenzträgern mit relativ großen Reaktionsflächen (Druchmesser > 1 cm), bevorzugt reines Wasser oder eine Waschlösung mit relativ geringen Konzentrationen der oben genannten Substanzen (Puffer, Detergenz, sonstige Zusätze) verwendet, um eine möglichst rückstandsfreie Absaugung zu erzielen.

Bevorzugt wird die wässrige Flüssigkeit über die Vorbeschichtung geleitet und dann vollständig entfernt, z.B. abgesaugt, so daß die Vorbeschichtung trocken zurückbleibt. Bevorzugt wird eine Vorbeschichtung verwendet, die hydrophobe Eigenschaften aufweist.

Auf die gewaschene Vorbeschichtung wird dann eine zweite Beschichtung aufgebracht, umfassend Rezeptormoleküle, die mit der Vorbeschichtung bindefähig sind, und zwar in Form von räumlich begrenzten Flächen auf dem Reagenzfeld. Die Rezeptormoleküle enthalten bevorzugt den zweiten Partner des Bindepaares, der eine hochaffine Wechselwirkung, z.B. eine immunologische Reaktion, eine Streptavidin/Avidin-Wechselwirkung oder ähnliches oder auch eine kovalente Bindung mit dem als Vorbeschichtung aufgebrachten ersten Partner des Bindepaares eingehen kann. So kann beispielsweise als Vorbeschichtung Streptravidin oder Avidin aufgebracht werden und das Rezeptormolekül enthält dann einen Biotinanteil. Bevorzugt erfolgt die Bindung der Rezeptormoleküle an die Vorbeschichtung mittels hochaffiner Wechselwirkungen mit einer Gleichgewichtskonstante K_{M} ≥ 10⁸ l/mol. Durch das Aufbringen der zweiten Beschichtung auf die gewaschene Vorbeschichtung erhält man reproduzierbare, gleichförmige Konturen und Größen der Testflächen.

Dieser Effekt wird durch die beigefügten Figuren weiter erläutert, worin
- Figur 1: miniaturisierte Testflächen zeigt, die auf herkömmliche Weise ohne Waschschritt hergestellt wurden,
- Figur 2: miniaturisierte Testflächen zeigt, die gemäß dem erfindungsgemäßen Verfahren hergestellt worden sind und
- Figur 3: die Wirkung des Waschschritts veranschaulicht, wobei Figur 3a ein mit Wasser gefülltes unbeschichtetes Enzymun-Teströhrchen (das ist ein unbeschichtetes Polystyrolröhrchen) zeigt, Figur 3b ein mit Wasser gefülltes Streptavidin beschichtetes Enzymun-Teströhrchen (das ist ein Streptavidin-beschichtetes Polystyrolröhrchen, Boehringer Mannheim, Best.-Nr. 1144553, "Enzymun-Test Streptavidin Tube") zeigt und Figur 3c ein Streptavidin beschichtetes und anschließend gewaschenes und getrocknetes Enzymun-Teströhrchen, in das Wasser gefüllt wurde, zeigt.

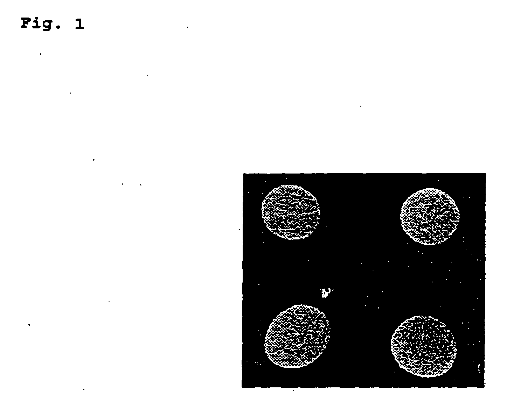

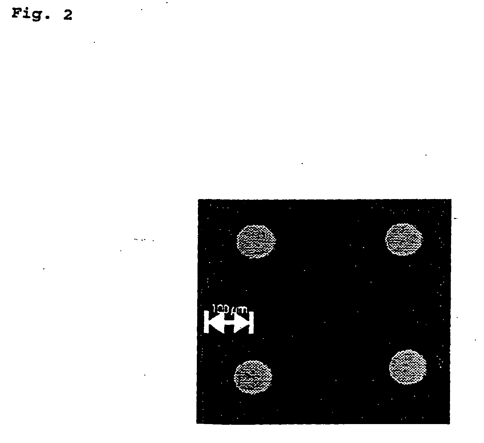

Ein Vergleich der in Figur 1 und Figur 2 gezeigten Testflächen demonstriert deutlich den durch den Waschschritt erhaltenen Vorteil, nämlich daß reproduzierbare, gleichmäßige Spotkonturen von Testflächen auf einem Reagenzfeld erhalten werden können.

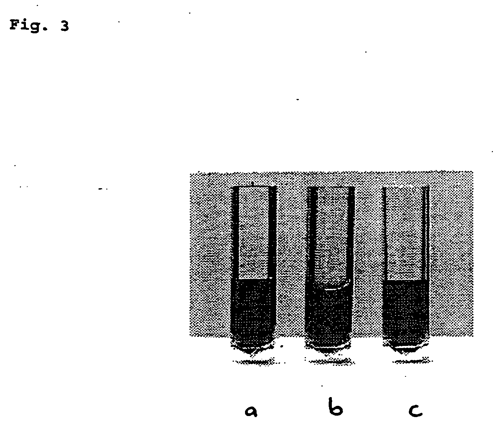

Befüllt man ein unbeschichtetes Enzymun-Teströhrchen mit Wasser, wie in Figur 3a gezeigt, so bildet sich eine völlig waagrechte Wasser-Luft-Grenzfläche aus. Dies bedeutet eine geringe Benetzung der Wand mit Wasser. Befüllt man hingegen ein mit Streptavidin vorbeschichtetes Enzymun-Teströhrchen mit Wasser, so ergibt sich eine stark gekrümmte Wasser-Luft-Grenzfläche, wie in Figur 3b gezeigt. Die Vorbeschichtung führt zu einer hohen Wandbenetzung. Befüllt man ein Teströhrchen, welches zunächst mit Streptavidin vorbeschichtet, dann intensiv mit einer wässrigen Flüssigkeit gewaschen und anschließend getrocknet wurde, so ergibt sich wiederum eine waagrechte Wasser-Luft-Grenzfläche, wie in Figur 3c gezeigt. Die Vorbeschichtung zeigt nach dem Waschschritt hydrophobe Eigenschaften, weshalb eine geringe Benetzung der Wand mit Wasser erfolgt.

Als fester, nicht poröser Träger kann erfindungsgemäß jedes feste, nicht poröse Material verwendet werden. Bevorzugt besteht der Träger aus Metall, Glas oder einem Kunststoff, insbesondere aus Polystyrol.

Die zweite Beschichtung wird bevorzugt in Form von räumlich begrenzten Flächen mit einem Durchmesser von 10 µm bis 10 mm aufgebracht. Da sich das erfindungsgemäße Verfahren insbesondere zur Herstellung von miniaturisierten Testformaten eignet, wird die zweite Beschichtung bevorzugt in Form von räumlich begrenzten Flächen mit einem Durchmesser von 10 µm bis 500 µm, besonders bevorzugt von 20 µm bis 200 µm aufgebracht. Das Aufbringen der zweiten Beschichtung kann mit bekannten Verfahren erfolgen. Zweckmäßigerweise wird die zweite Beschichtung in einer wässrigen Lösung in Form von Mikrotröpfchen mit einem Volumen zwischen 0,00001 bis 1 µl aufgebracht, z. B. mit Hilfe eines Flüssigkeitsstrahlverfahrens. Es ist aber erfindungsgemäß auch möglich, größere Reaktionsgefäße, wie z. B. Mikrotiterplatten oder Küvetten zu beschichten.

Bevorzugt wird das Reagenzfeld des festen Trägers vollflächig vorbeschichtet, wonach auf diese vollflächige Vorbeschichtung die zweite Beschichtung in Form von räumlich begrenzten Testflächen aufgebracht wird. Es ist aber auch möglich, bereits die Vorbeschichtung in Form von Spots aufzubringen, was jedoch in einen höheren Aufwand resultiert, da die zweite Beschichtung dann genau auf die vorbeschichteten Flächen positioniert werden muss.

Es ist weiterhin möglich, die Rezeptormoleküle enthaltende Beschichtungslösung direkt auf den festen, nicht porösen Träger aufzubringen.

Die Reproduzierbarkeit des erfindungsgemäßen Verfahrens ermöglicht es, Testsysteme bereitzustellen, die mehrere gleiche oder verschiedene Testflächen, insbesondere in miniaturisierter Form enthalten, so dass sowohl die Menge des benötigten Analyten als auch die Analysezeiten deutlich verringert werden können. Es ist deshalb bevorzugt, mehrere räumlich begrenzte Flächen der zweiten Beschichtung auf einen Träger aufzubringen. Die Flächen können dabei alle die gleichen Rezeptormoleküle aufweisen. In diesem Fall kann eine Vielzahl von verschiedenen Proben gleichzeitig auf einem Testträger untersucht werden. Wenn mindestens zwei der Flächen jeweils unterschiedliche Rezeptormoleküle aufweisen, können in einer Probe mehrere Analyten parallel bestimmt werden. Bei solchen Systemen ist es besonders wichtig, räumlich scharf begrenzte Testflächen zu erzeugen und jegliches Verschmieren der Rezeptormoleküle zu vermeiden. Nur so kann sichergestellt werden, dass tatsächlich der gewünschte Analyt, der an eine spezifische Testfläche bindefähig ist, bestimmt wird und nicht eine Kontamination benachbarter Flächen mit Rezeptormolekülen anderer Testflächen zu falschen Resultaten führt.

Ein weiterer Gegenstand der Erfindung ist eine Festphase, die durch das zuvor beschriebene Verfahren erhältlich ist, umfassend optional eine Vorbeschichtung auf einen festen Träger und eine an die Vorbeschichtung in Form von mehreren, kreisförmigen räumlich begrenzten Flächen gebundene zweite Beschichtung, welche dadurch gekennzeichnet ist, dass die zweite Beschichtung an die Vorbeschichtung gebundene Rezeptormoleküle umfasst und dass der Durchmesser der Flächen der zweiten Beschichtung 10 µm bis 10 mm beträgt und von Fläche zu Fläche weniger als 10%, bevorzugt um weniger als 5% und besonders bevorzugt um weniger als 2,5% variiert. Besonders bevorzugt wird diese Festphase in einem miniaturisierten Testsystem verwendet, wobei der Durchmesser der Testflächen 10 bis 500 µm, bevorzugt 20 bis 200 µm beträgt.

Weiterhin umfasst die Erfindung die Verwendung einer solchen Festphase zum Nachweis eines oder mehrerer Analyten, insbesondere in Bindungsassays, wie etwa einem Immunoassay, einem Nukleinsäurehybridisierungsassay usw.

Ebenfalls offenbart wird ein Verfahren zur Verbesserung der Gleichmässigkeit räumlich begrenzter Beschichtungsflächen auf einem festen, insbesondere nicht porösen Träger, umfassend die Schritte:
(a) Aufbringen einer Vorbeschichtung auf ein Reagenzfeld des festen Trägers,
(b) Waschen des vorbeschichteten Trägers mit einer wässrigen Flüssigkeit und
(c) Aufbringen einer zweiten Beschichtung, die mit der Vorbeschichtung bindefähig sind, auf den vorbeschichteten Träger in Form von räumlich begrenzten Flächen auf dem Reagenzfeld.

Ein Problem, das häufig bei der Herstellung oder/und Verwendung von Festphasen für Bindeassays auftritt, ist das Verschmieren von Rezeptormolekülen ausserhalb der dafür vorgesehenen, räumlich begrenzten Flächen beim Aufbringen einer Nachbeladelösung.

Ein zweiter Aspekt der Erfindung betrifft deshalb ein Verfahren zur Vermeidung des Verschmierens von räumlich begrenzten Festphasen, wobei die erneute Bindung von eluierten Rezeptormolekülen aus den räumlich begrenzten Festphasen bei Aufbringen einer Nachbeladungslösung verhindert wird, umfassend die Schritte:
(a) Aufbringen einer Rezeptormoleküle enthaltenden Beschichtungslösung auf vorbestimmte begrenzte Bereiche des festen Trägers,
(b) Binden der Rezeptormoleküle in den räumlich begrenzten Flächen,
(c) Trocknen der Beschichtungslösung und
(d) Aufbringen einer Nachbeladelösung, wobei zu der Beschichtungslösung ein Eluationsverzögerer zugegeben wird, wobei der Elutionsverzögerer ausgewählt ist aus Saccariden, Polyvinylpyrrolidon, Dextran, Gelatine oder Zellulosederivaten, wobei die Nachbeladelösung eine Blockierungssubstanz enthält.

Ebenfalls offenbart wird ein Verfahren zur Vermeidung des Verschmierens von räumlich begrenzten Festphasen, welche durch Aufbringen einer Rezeptormoleküle enthaltenden Beschichtungslösung auf vorbestimmte begrenzte Bereiche eines festen Trägers und anschließendes Behandeln mit einer Nachbeladelösung erzeugt werden, umfassend mindestens eine der Massnahmen:
(a) Aufbringen der Beschichtungslösung, auf eine Weise, so dass eine im wesentliche quantitative Bindung der Rezeptormoleküle innerhalb der vorbestimmten begrenzten Bereiche erfolgt oder/und.
(b) Verhinderung der erneuten Bindung von eluierten Rezeptormolekülen aus den vorbestimmten begrenzten Bereichen bei Aufbringung einer Nachbehandlungslösung.

Überraschenderweise wurde festgestellt, dass unter Verwendung mindestens einer dieser Maßnahmen das Verschmieren von räumlich begrenzten Festphasen vermieden werden kann, was in scharf begrenzten Testflächen mit reproduzierbaren Konturen und Größen resultiert. Dieses Verfahren kann sowohl für einlagige als auch für mehrlagige Beschichtungen verwendet werden.

Rezeptormoleküle werden im Allgemeinen in wässriger Lösung in Form kleiner Tröpfchen auf einen festen Träger aufgetragen, der gegebenenfalls vorbeschichtet sein kann. Aus der Lösung heraus diffundieren die Rezeptormoleküle dann an den festen Träger bzw. an die Vorbeschichtung und binden an diese. Diese Bindungswechselwirkung kann eine hochaffine Wechselwirkung wie etwa eine immunologische Reaktion oder eine Streptavidin/Avidin-Biotin-Wechselwirkung oder eine kovalente Bindung sein. Bei herkömmlichen Festphasensystemen des Standes der Technik bewirken Nachfolgeprozesse nach dem Aufbringen der Beschichtung z. B. bei der Versuchsdurchführung zur Analyse eines Analyten oder das Aufbringen einer Nachbeladelösung eine Eluation ungebundener oder nicht genügend gebundener Moleküle von der Festphase.

Eine spezifisch beschichtete Festphase wird üblicherweise mit einer Nachbeladelösung behandelt, deren Aufgabe es ist, unspezifische Bindestellen abzublocken und die Rezeptormoleküle gegen äußere Einflüsse zu stabilisieren. Dies ist notwendig, da die Testflächen des Reagenzfeldes einer Festphase zumeist nicht vollständig zu 100% belegt sind. Freibleibende Stellen des Trägers bzw. der Vorbeschichtung stellen jedoch unspezifische Bindungsstellen dar, an die andere Probenbestandteile als der gewünschte Analyt binden können.

Weiterhin enthält eine Beschichtung mit Rezeptormolekülen zumeist auch einen geringen Anteil geschädigter Moleküle, an die andere Probenbestandteile als der erwünschte Analyt unspezifisch binden können. Durch Aufbringen einer Nachbeladelösung, welche beispielsweise Inertproteine enthält, werden solche Stellen blockiert, so daß die spätere Anlagerung von Probenbestandteilen während der Analyse verhindert wird. Zudem dient eine Nachbeladelösung oftmals auch zur Stabilisierung der Rezeptormoleküle bzw. der Beschichtung gegen äußere Einflüsse wie etwa Temperatur oder Feuchtigkeit. In vielen Ausführungsformen von Analysesystemen, wie z.B. Mikrotiterplatten oder ES-Tubes liegen Verfahrensabläufe vor, in denen die Nachbeladelösung sowohl blockierende als auch stabilisierende Aufgaben erfüllt.

Während die Verwendung einer Nachbeladelösung für viele Testformate notwendig ist, ist damit zugleich oftmals das Verschmieren der zuvor aufgebrachten Rezeptormoleküle verbunden. Das Aufbringen der Nachbeladelösung bewirkt eine Elution ungebundener oder ungenügend gebundener Rezeptormoleküle. Diese eluierten Rezeptormoleküle können dann unerwünschterweise an beliebigen noch zugänglichen Stellen des festen Trägers bzw. der Vorbeschichtung binden und führen somit zu einem Verschmieren von zuvor räumlich begrenzten Festphasen, da sich die Rezeptormoleküle auch außerhalb des Primärauftrags anlagern können. Ein solches Verschmieren ist insbesondere dann von großem Nachteil, wenn auf einem Träger mehrere räumlich begrenzte Flächen aufgebracht sind, die jeweils unterschiedliche Rezeptormoleküle aufweisen. Die Elution von Rezeptörmölekülen und Bindung von eluierten Rezeptormolekülen an anderen Stellen der Festphase kann zu einer Vermischung der Rezeptoren in den Testflächen führen, so daß kein eindeutiges Analyseergebnis erhalten wird. Dieses Problem tritt insbesondere bei miniaturisierten Testformaten auf. Auch bei Tests, bei denen keine Nachbeladelösung verwendet wird, treten die gleichen Probleme auf, nämlich spätestens zu dem Zeitpunkt, an dem eine Probenlösung aufgebracht wird.

Überraschenderweise wurde nun gefunden, daß durch Aufbringen einer Beschichtungslösung, wobei eine im wesentlichen quantitative Bindung der Rezeptormoleküle innerhalb vorbestimmter Bereiche erfolgt, das Verschmieren der räumlich begrenzten Festphasen vermieden werden kann. Durch eine nahezu 100%ige Bindung der Rezeptormoleküle kann deren Elution verhindert werden. Bevorzugt werden die Rezeptormoleküle zu mehr als 90 %, besonders bevorzugt zu mehr als 95 % und am meisten bevorzugt zu mehr als 99 % gebunden. Eine im wesentlichen quantitative Bindung der Rezeptormoleküle wird bevorzugt durch die Verwendung einer Beschichtungslösung erzielt, die eine Konzentration an Rezeptormolekülen aufweist, die geringer als die Grenzkonzentration ist, die ausreicht um den vorbestimmten Bereich vollständig zu belegen. Wenn die Beschichtungslösung weniger Rezeptormoleküle enthält als an der Testfläche bindefähig sind, so werden alle aufgebrachten Rezeptormoleküle in der Testfläche gebunden und es liegen keine ungebundenen oder ungenügend gebundenen Rezeptormoleküle vor, die später, beispielsweise durch eine Nachbeladelösung, eluiert werden können. Grenzkonzentrationen für verschiedene Rezeptormoleküle sind in Tabelle 4 dargestellt.

**Tabelle 4 Grenzkonzentrationen unterschiedlicher Beschichtungsmoleküle**

| **Rezeptormolekül** | **Durchmesser 10 *µ*m** | **Durchmesser 10 *µ*m** | **Durchmesser 1mm** |
|---|---|---|---|
| Antikörper | 2,5 mg/ml | 250 µg/ml | 25 µg/ml |
| Antigen (z.B. p24) | 600 µg/ml | 60 µg/ml | 6 µg/ml |
| Peptid (MG 3000) | 150 µg/ml | 15 µg/ml | 1,5 µg/ml |

Bevorzugt wird eine Beschichtungslösung verwendet, die eine Konzentration an Rezeptormolekülen enthält, die kleiner als die Grenzkonzentration ist, bevorzugt kleiner als 50% und besonders bevorzugt kleiner als 25% der Grenzkonzentration.

Weiterhin ist es bevorzugt, stark bindende Rezeptormoleküle zu verwenden, beispielweise vernetzte Antikörper anstelle von monomeren Antikörpern. Insbesondere durch das Vernetzen von Antikörpern zu Polymeren mit einem Molekulargewicht von > 1,2 Millionen Dalton kann ein Verschmieren verhindert werden, da gefunden wurde, daß hochvernetzte Proteine sehr gut an feste Träger, insbesondere an Kunststoffoberflächen binden. Besonders bevorzugt wird als Rezeptormolekül rekombinantes HBc-Antigen verwendet, wobei durch Selbstaggregation Makromoleküle im Bereich von mehreren Millionen Dalton gebildet werden, sowie tRSA-Konjugate, wie etwa tRSA-Biotin, tRSA-Streptavidin oder tRSA-Anti-TSH-Antikörper. In allen Fällen wurde eine nahezu 100%-ige Bindung und damit keinerlei Verschmierung von Rezeptormolekülen außerhalb der Zielfläche gefunden.

Die Rezeptormoleküle können unmittelbar auf den festen Träger oder auf eine auf den festen Träger aufgebrachte Vorbeschichtung aufgebracht werden. Im Falle einer Vorbeschichtung ist es bevorzugt, Rezeptormoleküle zu verwenden, die eine hohe Bindungsaffinität von mindestens 10⁸ l/mol an die Vorbeschichtung aufweisen.

Weiterhin wurde gefunden, daß ein Verschmieren von räumlich begrenzten Festphasen durch Zugabe eines Eluationsverzögerers zu der Beschichtungslösung verhindert werden kann. Unter Eluationsverzögerern werden Substanzen verstanden, die nach Eintrocknen der Beschichtungslösung einen Überzug über der Testfläche bilden und so eine unmittelbare Eluierung ungebundener oder nicht genügend gebundener Rezeptormoleküle bei Zugabe einer Nachbeladelösung oder Probelösung verhindern oder verzögern. Geeignete Eluationsverzögerer sind beispielsweise Saccharide wie etwa Saccharose, PVP (Polyvinylpyrrolidon) oder Dextran, welches bevorzugt ein Molekulargewicht von 10 000 bis 100 000 Da aufweist. Weitere geeignete Eluationsverzögerer sind Gelatine und Zellulosederivate, insbesondere Methylcellulose, die bevorzugt dann eingesetzt werden, wenn die Rezeptormoleküle mit einer zuvor aufgebrachten Vorbeschichtung reagieren. Durch die Zugabe eines Eluationsverzögerers wird die Eluierung ungebundener Rezeptormoleküle wenigstens solange hinausgezögert, bis alle freien Stellen auf den festen Träger durch geeignete Maßnahmen blockiert worden sind, so daß eluierte Rezeptormoleküle nicht mehr binden können und ausgewaschen werden.

In einem zweiten Aspekt wird das erneute Binden von eluierten Rezeptormolekülen aus vorbestimmten Bereichen bei Aufbringen einer Nachbeladelösung verhindert. Dies wird bevorzugt durch Zugabe einer Blockierungssubstanz zur Nachbeladelösung erreicht. Hierbei ist es wesentlich, dafür zu sorgen, daß die Bindung der Blockierungssubstanzen möglichst schnell erfolgt. Dies kann dadurch erreicht werden, daß man die Nachbeladelösung mit hohen Konzentrationen von ≥ 0,5 Gew.-%, bevorzugt ≥ Gew.-% an einer Blockierungssubstanz versetzt, beispielsweise mit 1 Gew.-% RSA (Rinderserumalbumin), Crotein C, Kasein etc. Weitere geeignete Blockierungssubstanzen sind z.B. Pepton, Rinder IgG, Kaninchen IgG. Collagen, Gelatine, Polyethylenglykol und Detergenzien, wie etwa Tween 20.

Die Bindungskinetik der Blockierungssubstanz, insbesondere von Blockproteinen kann auch durch geeignete Pufferzusätze, wie etwa 3% MgCl₂ verbessert werden. Wenn die Beschichtungslösung nicht direkt auf den festen Träger, sondern auf eine Vorbeschichtung aufgebracht wird, werden Blockierungssubstanzen gewählt, die spezifisch mit der Vorbeschichtung reagieren. Anstelle bzw. zusätzlich zu unspezifischen Blockierungssubstanzen, wie etwa Blockproteinen, kann durch solche spezifischen Blocksubstanzen, die mit der Vorbeschichtung spezifisch bindefähig sind, die Blockierungswirkung noch verbessert werden. Hierzu wird beispielsweise auf eine Streptavidinvorbeschichtung eine biotinylierte Rezeptormoleküle enthaltende Lösung aufgebracht. Nach dem Eintrocknen wird darauf eine Biotinlösung aufgetragen, durch die die noch freien Streptavidin-Bindestellen abgesättigt werden.

Schließlich ist es erfindungsgemäß auch möglich, das Verschmieren von räumlich begrenzten Festphasen durch Aufbringen der Nachbeladelösung innerhalb einer geringen Zeitdauer zu verhindern, so daß eluierte Rezeptormoleküle unmittelbar stark vedünnt werden. Die maximale Zeitdauer beträgt dabei im Allgemeinen 500 msec, bevorzugt 250 msec und besonders bevorzugt 50 msec. Hier ist es von Vorteil, für eine schnelle Durchmischung zu sorgen, um in der Nachbeladelösung Bereiche mit hoher lokaler Konzentration an eluierten Rezeptormolekülen zu verhindern. Eine effiziente Mischung ist beispielsweise dadurch zu erreichen, daß die Nachbeladelösung mit einer möglichst hohen Strömungsgeschwindigkeit aufgebracht wird, wodurch ein möglichst kurzer zeitlicher Kontakt zwischen Nachbeladelösung und Testflächen gewährleistet wird.

Besonders bevorzugt werden mehrere der angegebenen Maßnahmen in Kombination eingesetzt, um ein Binden von eluierten Rezeptormolekülen außerhalb der vorgesehenen Testflächen zu verhindern. Als besonders vorteilhaft hat es sich erwiesen, der Beschichtungslösung einen Eluationsverzögerer und der Nachbeladelösung eine Blockierungssubstanz zuzusetzen und gleichzeitig für eine schnelle Durchmischung der Nachbeladelösung zu sorgen.

Da dieses Verfahren insbesondere bei miniaturisierten Testformaten vorteilhaft ist, weisen die räumlich begrenzten Festphasen bevorzugt einen Durchmesser von 10 bis 500 µm, besonders bevorzugt von 20 bis 200 µm auf.

Die Erfindung umfaßt auch eine durch das oben beschriebene Verfahren zur Vermeidung des Verschmierens von räumlich begrenzten Festphasen erhältliche Festphase, welche durch Aufbringen einer Rezeptormoleküle enthaltenden Beschichtungslösung auf vorbestimmte Bereiche eines festen Trägers und anschließendes Behandeln mit einer Nachbeladelösung erzeugt wird, wobei die Rezeptormoleküle in räumlich begrenzten Flächen angeordnet sind, welche dadurch gekennzeichnet ist, daß sich ≥ 80%, insbesondere ≥ 90% und besonders bevorzugt ≥ 95% der Rezeptormoleküle innerhalb der räumlich begrenzten Flächen befinden. Eine solche Festphase ermöglicht zuverlässige und reproduzierbare Analyseergebnisse. Eine solche Festphase kann zum Nachweis eines oder mehrerer Analyten verwendet werden.

Eine weitere häufige Ursache für falsche Ergebnisse bei Bindungsassays sind unspezifische Bindungen, d.h. Bindungen von Probenbestandteilen außer dem gewünschten Analyten, an mit Streptavidin oder Avidin beschichtete Festphasen.

Die vorliegende Erfindung betrifft deshalb auch ein Verfahren zur Verringerung der unspezifischen Bindung an eine mit Streptavidin oder Avidin beschichtete Festphase, umfassend die Schritte
(a) Aufbringen eines aus einem Makromolekül und Biotin bestehenden Konjugats und
(b) Aufbringen von monomerem Streptavidin oder Avidin auf die Festphase.

Überraschenderweise wurde festgestellt, daß durch dieses Verfahren eine deutliche Verringerung von unspezifischer Bindungen an eine mit einer Streptavidin oder Avidin beschichtete Festphase erreicht werden kann. Die Verwendung von monomerem Streptavidin verringert insbesondere die unspezifische Bindung von Konjugaten und anderen Probenbestandteilen an die Festphase erheblich.

Bevorzugt wird das aus einem Makromolekül, wie etwa tRSA, und Biotin bestehende Konjugat als wässrige Lösung in Form von Mikrotröpfchen auf einen festen Träger aufgebracht. Bevorzugt wird dabei ein Konjugat verwendet, das ein Makromolekül und Biotin im Verhältnis 1:1 bis 1:3, besonders bevorzugt 1:1 bis 1:2 umfaßt. Durch eine solche niedrige Biotinylierungs-Stöchiometrie des Klettproteins kann die unspezifische Bindung, insbesondere von Konjugaten weiter wesentlich verringert werden.

Das erfindungsgemäße Verfahren zur Verringerung der unspezifischen Bindung an eine mit Streptavidin oder Avidin beschichtete Festphase eignet sich insbesondere auch für miniaturisierte Testformate, wobei die Festphase bevorzugt einen Durchmesser von ≤ 10 mm, besonders bevorzugt ≤ 5 mm aufweist.

Ein weiterer Gegenstand der Erfindung ist eine Festphase, erhältlich durch das oben beschriebene Verfahren, welche dadurch gekennzeichnet ist, daß sie eine Vorbeschichtung eines aus einem Makromolekül und Biotin bestehenden Konjugats und darauf eine Schicht von monomerem Streptavidin oder Avidin aufweist. Eine solche Festphase wird insbesondere zum Nachweis eines Analyten verwendet.

Die Erfindung wird durch die folgenden Beispiele weiter erläutert:

### Beispiele

### Beispiel 1 Herstellung von Spots auf einer streptavidinbeschichteten Oberfläche

Die Testflächen wurden mit einer Beschichtungslösung beaufschlagt, die eine hochmolekulare Verbindung RSA-Streptavidin mit einer Partikelgröße von ca. 100 nm enthielt. Nach einer Reaktionszeit von 15 min wurde die Beschichtungslösung abgesaugt und die Testflächen mit einer Lösung bestehend aus 0,05 % NaCI, 0,2% Saccharose und 0,05% RSA geblockt. Bei der in Fig. 1 gezeigten Testfläche wurde nach einer Inkubationszeit der Vorbeschichtung von 5 min nur trocken gesaugt, während bei den in Fig. 2 gezeigten Testflächen ein erfindungsgemäßer Waschschritt mit einem wäßrigen Medium durchgeführt wurde und anschließend trockengesaugt wurde.

### Beispiel 2

3,7 g Tris (Tris(hydroxymethyl)-aminomethan und 4,75 g MES (2-Morpholinoethansulfonsäure) werden in 5 l Wasser gelöst. Mit 5 ml dieses Waschpuffers wird ein vorbeschichtetes Reaktionsgefäß mit einem Nennvolumen von 50 µl überströmt. Anschließend wird das Reaktionsgefäß mit einer oder mehreren Absaugnadeln trockengesaugt. Durch Aufbringen einer zweiten Beschichtung auf die gewaschene und getrocknete Vorbeschichtung erhält man räumlich eng begrenzte Testflächen, wie sie beispielsweise in Figur 2 dargestellt sind.

### Beispiel 3

Auf einen festen Träger wird eine Streptavidinvorbeschichtung aufgebracht. Auf diese Beschichtung wird eine Beschichtungslösung mit mono-biotinylierte Antikörper in Form kleiner Tröpfchen aufgegeben. Nachdem die Tröpfchen eingetrocknet sind, wird darauf eine Lösung gegeben, die 3 mg/ml Biotin in 50 mM K₂HPO₄ enthält. Nach einigen Sekunden wird die Biotinlösung entfernt und eine Lösung mit 1% RSA (Rinderserumalbumin) und 2% Saccharose aufgebracht, welche nach wenigen Sekunden ebenfalls entfernt wird. Anschließend wird das System getrocknet. Auf diese Weise werden räumlich scharf begrenzte Festphasen erhalten, ohne Verschmierung von Rezeptormolekülen außerhalb der gewünschten Reaktionsfläche.

### Beispiel 4

Es wurden unspezifische Bindungen auf verschiedenen Streptavidin-Festphasen untersucht. Die Ergebnisse sind in Tabelle 5 zusammengestellt.

**Tabelle 5**

| **Beschichtungs-Typ** | **TSH-Konjugat** | **p24-Konjugat** | **Human-IgG** |
|---|---|---|---|
| tRSA-Streptavidin | 24 | 31 | 105 |
| tRSA-Biotin + polymeres Streptavidin | 3 | 71 | 63 |
| tRSA-Biotin + monomeres Streptavidin | 0 | 4 | 38 |

Wie deutlich zu sehen ist, kann durch die erfindungsgemäße Kombination aus Konjugat und monomerem Streptavidin eine deutliche Verringerung der unspezifischen Bindung erzielt werden.

### Beispiel 5 Direktbeschichtung

Zunächst wird ein Polyhapten durch Kopplung mehrerer identischer Peptide an RSA hergestellt. Das Polyhapten wird auf 50µg/ml in einen Puffer umfassend 5 mM Mes, 5 mM Tris und 1 % Saccharose gelöst. Anschließend werden Tröpfchen mit einem Volumen von 150 pl auf einen Polystyrolträger aufgebracht, für ca. 5 s inkubiert und für etwa 60 s getrocknet. Anschließend wird eine Blocklösung bestehend aus 1% RSA und 2% Saccharose aufgebracht und für 10 s inkubiert. Anschließend wird abgesaugt und eine Blocklösung, bestehend aus 1% RSA und 2% Saccharose zugegeben. Anschließend wird abgesaugt und getrocknet.

### Beispiel 6 Streptavidin-Vorbeschichtung

Zunächst wird ein biotinyliertes Makromolekül (z.B. thermisch aggregiertes RSA) in einer Konzentration von 100/µg/ml in PBS auf einen Polystyrolträger mit 50 µl Nennvolumen aufgebracht und 5 min inkubiert. Anschließend wird abgesaugt und eine Streptavidinlösung mit einer Konzentration von 100µg/ml in PBS zugegeben. Nach 5 min Inkubation erfolgt ein Waschschritt mit anschließendem Trockensaugen.

Unterschiedliche, biotinylierte DNA-Fangsonden (18-mer) werden in einer Konzentration von 1 µM in einen Puffer aus 5 mM Mes, 5 mM Tris, 1% Saccharose und 0,5 mg/ml RSA gelöst. Anschließend werden Tröpfchen mit einem Volumen von ca. 150 pl in Form eines Arrays auf einen Polystyrolträger aufgebrächt. Nach einer Inkubation für ca. 5 s und Trocknen für ca. 60 s wird eine Lösung bestehend aus 3 mg/ml Biotin in 50 mM Dikaliumphosphat zugegeben und für 2 s inkubiert. Anschließend wird abgesaugt und eine Blocklösung, bestehend aus 1% RSA und 2% Saccharose zugegeben, abgesaugt und getrocknet.

### Beispiel 7 Vorbeschichtung mit Anti-Maus-Antikörpern

PAK <Maus-F_{cy} >S lgG werden in einer Konzentration von 30 µg/ml in einem Puffer umfassen 40 mM Kaliumphosphat bei einem pH-Wert von 7,4 in 0,9% Natriumchlorid gelöst. Diese Lösung wird in ein Reaktionsgefäß mit 50 µl Nennvolumen aufgebracht. Nach 15 minütiger Inkubation und anschließendem Absaugen erfolgt die Zugabe einer Blocklösung bestehend aus 1% RSA und 2% Saccharose. Nach 5 min Inkubation erfolgt erfindungsgemäß ein Waschschritt mit anschließendem Trockensaugen.

Ein Fusionsprotein (Maus-F_{cy} + CD28) wird in einem Puffer umfassend 5 mM Mes, 5 mM Tris, 1% Saccharose und 0,5 mg/ml RSA auf 100/µg/ml gelöst. Es werden Tröpfchen mit einem Volumen von ca. 150 pl auf den zuvor beschriebenen, beschichteten Polystyrolträger aufgebracht und für ca. 5 s inkubiert. Nach Trocknen für ca. 60 s erfolgte die Zugabe einer Blocklösung bestehend aus 50 mM Kaliumphosphatpuffer, pH 7,4, 100 µg/ml Maus lgG, 1 mg/ml RSA. Nach Inkubation für 10 s wird abgesaugt und eine Blocklösung bestehend aus 1% RSA, 2% Saccharose zugegeben, abgesaugt und getrocknet.

### Beispiel 8 Universelle Streptavidin-Testflächen

Ein Makromolekül von ca. 100 nm Partikelgröße, bestehend aus RSA und Streptavidin wird auf eine Konzentration von 500 µg/ml in 20 mM Phosphatpuffer pH 7,4 und 1% Saccharose gelöst. Tröpfchen mit einem Volumen von etwa 150 pl werden auf einen Polystyrolträger aufgebracht, für ca. 5 s inkubiert und für ca. 60 s getrocknet. Anschließend wird eine Lösung, bestehend aus 1 % RSA und 2% Saccharose zugegeben, für ca. 10 s inkubiert und abgesaugt. Dann wird eine Lösung bestehend aus 1% RSA, 2% Saccharose, 1 µg/ml biotinylierter Anti-TSH-Antikörper zugegeben, für 20 min inkubiert und abgesaugt. Schließlich wird eine Blocklösung bestehend aus 1% RSA und 2% Saccharose zugegeben, abgesaugt und getrocknet. Das Ergebnis ist eine hochpräzise, miniaturisierte TSH-spezifische Festphase.

### Beispiel 9 Testflächen für Anti-Rubella-Antikörper

Ein biotinyliertes Makromolekül (z.B. thermisch aggregiertes RSA) wird in einer Konzentration von 100 µg/ml in PBS auf einen Polystyrolträger mit 50 µl Nennvolumen aufgebracht, 5 min inkubiert und abgesaugt. Anschließend wird eine Streptavidinlösung mit einer Konzentration von 100µg/ml in PBS zugegeben. Nach 5 min Inkubation erfolgt erfindungsgemäß ein Waschschritt mit anschließendem Trockensaugen.

Ein biotinylierter Anti-Rubella-Antikörper wird auf eine Konzentration von 50 µg/ml in einen Puffer aus 5 mM Mes, 5 mM Tris, 1% Saccharose und 0,5 mg/ml RSA gelöst. Tröpfchen mit einem Volumen von ca. 150 pl werden auf dem zuvor beschriebenen Träger aufgebracht. Es erfolgt eine Inkubation für ca. 5 s und ein Trocknen für ca. 60 s. Anschließend wird eine Lösung bestehend aus 3 mg/ml Biotin in 50 mM Dikaliumphosphat zugegeben, für 2 s inkubiert und abgesaugt. Dann wird eine Lösung, bestehend aus 0,1% Tween 20, 1% RSA, 3 U/ml Virus-Lysat (Rubella-Virus) in PBS zugegeben, für 20 min Inkubiert und dann abgesaugt. Anschließend wird eine Blocklösung, bestehend aus 1% RSA und 2% Saccharose zugegeben, abgesaugt und getrocknet.

## Patentansprüche

1. Verfahren zum Aufbringen von Rezeptormolekülen enthaltenden räumlich begrenzten Flächen auf einen festen, nicht porösen Träger, umfassend die Schritte:
(a) Aufbringen einer Rezeptormoleküle enthaltenden Beschichtungslösung auf vorbestimmte begrenzte Bereiche des festen Trägers,
(b) Binden der Rezeptormoleküle in den räumlich begrenzten Flächen,
(c) Trocknen der Beschichtungslösung und
(d) Aufbringen einer Nachbeladelösung, wobei zu der Beschichtungslösung ein Eluationsverzögerer zugegeben wird, wobei der Elutionsverzögerer ausgewählt ist aus Saccariden, Polyvinylpyrrolidon, Dextran, Gelantine oder Zellulosederivaten, wobei die Nachbeladelösung eine Blockierungssubstanz enthält.

2. Verfahren nach Anspruch 1, **dadurch gekennzeichnet, dass** die Beschichtungslösung in Form von räumlich begrenzten Flächen mit einem Durchmesser von 10 µm bis 10 mm aufgebracht wird.

3. Verfahren nach einem der vorhergehenden Ansprüche, **dadurch gekennzeichnet, dass** mehrere räumlich begrenzte Flächen aufgebracht werden, wobei mindestens zwei der Flächen jeweils unterschiedliche Rezeptormoleküle aufweisen.

4. Verfahren nach Anspruch 1, **dadurch gekennzeichnet, dass** die Nachbeladelösung ≥ 0,5 Gew.-% an einer Blockierungssubstanz enthält.

5. Verfahren nach einem der vorhergehenden Ansprüche, **dadurch gekennzeichnet, dass** die Nachbeladelösung innerhalb einer geringen Zeitdauer von maximal 500 msec aufgebracht wird.

6. Verfahren nach einem der vorhergehenden Ansprüche, weiterhin umfassend eine Vorbesschichtung, umfassend die Schritte
(a') Aufbringen einer Vorbeschichtung auf ein Reagenzfeld des festen Trägers,
(b') Waschen des vorbeschichteten Trägers mit einer wässrigen Flüssigkeit und
(c') Aufbringen der Rezeptormoleküle enthaltenden Beschichtungslösung auf vorbestimmte räumlich begrenzte Bereich des vorbeschichteten Trägers, wobei die Beschichtungslösung Rezeptormoleküle umfasst, die mit der Vorbeschichtung bindefähig sind.

7. Verfahren nach einem der Ansprüche 1 bis 6, **dadurch gekennzeichnet, dass** die Rezeptormoleküle enthaltende Beschichtungslösung direkt auf den festen, nicht porösen Träger aufgebracht wird.

8. Verfahren nach einem der vorhergehenden Ansprüche, **dadurch gekennzeichnet, dass** die Rezeptormoleküle enthaltende Beschichtungslösung in Form von Mikrotröpfchen aufgebracht wird.

9. Verfahren zur Vermeidung des Verschmierens von räumlich begrenzten Festphasen, wobei die erneute Bindung von eluierten Rezeptormolekülen aus den räumlich begrenzten Festphasen bei Aufbringen einer Nachbeladungslösung verhindert wird, umfassend die Schritte:
(a) Aufbringen einer Rezeptormoleküle enthaltenden Beschichtungslösung auf vorbestimmte begrenzte Bereiche des festen Trägers,
(b) Binden der Rezeptormoleküle in den räumlich begrenzten Flächen,
(c) Trocknen der Beschichtungslösung und
(d) Aufbringen einer Nachbeladelösung, wobei zu der Beschichtungslösung ein Eluationsverzögerer zugegeben wird, wobei der Elutionsverzögerer ausgewählt ist aus Saccariden, Polyvinylpyrrolidon, Dextran, Gelantine oder Zellulosederivaten, wobei die Nachbeladelösung eine Blockierungssubstanz enthält.

10. Verfahren nach Anspruch 9, **dadurch gekennzeichnet, dass** eine Beschichtungslösung aufgebracht wird, die eine Konzentration an Rezeptormolekülen von kleiner als 90 % der Grenzkonzentration enthält, die ausreicht, um den vorbestimmten Bereich vollständig zu belegen.

11. Festphase, erhältlich durch ein Verfahren nach einem der vorhergehenden Ansprüche, welche durch Aufbringen einer Rezeptormoleküle enthaltenden Beschichtungslösung auf vorbestimmte Bereich eines festen, nicht porösen Trägers und anschließendes Behandeln mit einer Nachbeladelösung erzeugt wird, wobei die Rezeptormoleküle in räumlich begrenzten Flächen angeordnet ist, **dadurch gekennzeichnet, dass** sich ≥ 80 % der Rezeptormoleküle innerhalb der räumlich begrenzten Flächen befinden.

12. Festphase nach Anspruch 11, **dadurch gekennzeichnet, dass** die Rezeptormoleküle enthaltenden vorbestimmten begrenzten Bereiche einen Durchmesser von 10 µm bis 10 mm aufweisen und von Fläche zu Fläche um weniger als 10 % variieren.

## Claims

1. Process for the application of spatially defined areas containing receptor molecules on a solid, non-porous support comprising the steps:
(a) application of a coating solution containing receptor molecules onto predetermined defined areas of the solid support,
(b) binding the receptor molecules in the spatially defined areas,
(c) drying the coating solution and
(d) applying a reloading solution,
wherein an elution retardant is added to the coating solution, the elution retardant being selected from saccharides, polyvinylpyrrolidone, dextran, gelatin or cellulose derivatives and the reloading solution contains a blocking substance.

2. Process according to claim 1, **characterized in that** the coating solution is applied in the form of spatially defined areas having a diameter of 10 µm to 10 mm.

3. Process according to one of the previous claims, **characterized in that** several spatially defined areas are applied where at least two of the areas each have different receptor molecules.

4. Process according to claim 1, **characterized in that** the reloading solution contains ≥ 0.5 % by weight of a blocking substance.

5. Process according to one of the previous claims, **characterized in that** the reloading solution is applied within a short time period of no more than 500 msec.

6. Process according to one of the previous claims, additionally comprising a precoating comprising the steps:
(a') applying a precoating to a reagent field of the solid support,
(b') washing the precoated support with an aqueous liquid and
(c') applying the coating solution containing receptor molecules to a predetermined spatially defined area of the precoated support, wherein the coating solution contains receptor molecules which are capable of binding to the precoating.

7. Process according to one of the claims 1 to 6, **characterized in that** the coating solution containing receptor molecules is applied directly to the solid, non-porous support.

8. Process according to one of the previous claims, **characterized in that** the coating solution containing receptor molecules is applied in the form of microdroplets.

9. Process for avoiding smearing of spatially defined solid phases in which eluted receptor molecules from the spatially defined solid phases are prevented from binding again when a reloading solution is applied comprising the steps:
(a) applying a coating solution containing receptor molecules to predetermined defined areas of the solid support,
(b) binding the receptor molecules in the spatially defined areas,
(c) drying the coating solution, and
(d) applying a reloading solution,
wherein an elution retardant is added to the coating solution, the elution retardant being selected from saccharides, polyvinylpyrrolidone, dextran, gelatin or cellulose derivatives and the reloading solution contains a blocking substance.

10. Process according to claim 9, **characterized in that** a coating solution is applied which contains a concentration of receptor molecules which is less than 90 % of the limiting concentration that is sufficient to completely occupy the predetermined area.

11. Solid phase obtainable by a process according to one of the previous claims, which is produced by applying a coating solution containing receptor molecules to a predetermined area of the solid, non-porous support and subsequent treatment with a reloading solution, wherein the receptor molecules are located in spatially defined areas, **characterized in that** ≥ 80 % of the receptor molecules are situated within the spatially defined areas.

12. Solid phase according to claim 11, **characterized in that** the predetermined defined areas containing receptor molecules have a diameter of 10 µm to 10 mm and vary from area to area by less than 10 %.

## Revendications

1. Procédé d'application de revêtement spatialement limités contenant des récepteurs sur un support solide non poreux, comprenant les étapes consistant à :
(a) appliquer une solution de revêtement contenant des récepteurs sur des zones limitées prédéterminées du support solide,
(b) lier les récepteurs dans les revêtement spatialement limités,
(c) sécher la solution de revêtement et
(d) appliquer une solution de post-charge, un retardateur d'élution étant ajouté à la solution de revêtement, le retardateur d'élution étant choisi parmi les saccharides, la polyvinylpyrrolidone, le dextran, la gélatine ou les dérivés cellulosiques, la solution post-charge contenant une substance de blocage.

2. Procédé selon la revendication 1, **caractérisé en ce que** la solution de revêtement est appliquée sous la forme de revêtements spatialement limités présentant un diamètre de 10 µm à 10 mm.

3. Procédé selon l'une quelconque des revendications précédentes, **caractérisé en ce que** plusieurs revêtements spatialement limitées sont appliqués, au moins deux des surfaces présentant des récepteurs différents.

4. Procédé selon la revendication 1, **caractérisé en ce que** la solution post-charge contient ≥ 0,5 % en poids d'une substance de blocage.

5. Procédé selon l'une quelconque des revendications précédentes, **caractérisé en ce que** la solution de post-charge est appliquée en l'espace de moins de 500 msec au maximum.

6. Procédé selon l'une des revendications précédentes, comprenant en outre un pré-revêtement, comprenant les étapes consistant à :
(a') appliquer un pré-revêtement sur un champ réactionnel du support solide,
(b') laver le support doté du pré-revêtement avec un liquide aqueux et
(c') appliquer la solution contenant les récepteurs sur une zone spatialement limitée prédéterminée du support pré-revêtu, la solution de revêtement comprenant des récepteurs qui peuvent se lier au pré-revêtement.

7. Procédé selon l'une des revendications 1 à 6, **caractérisé en ce que** la solution de revêtement contenant les récepteurs est appliquée directement sur le support solide non poreux.

8. Procédé selon l'une des revendications précédentes, **caractérisé en ce que** la solution de revêtement contenant les récepteurs est appliquée sous la forme de micro-gouttelettes.

9. Procédé pour éviter l'endommagement de phases solides limitées spatialement, la nouvelle liaison des récepteurs élués des phases solides limitées spatialement étant empêchée par application d'une solution de post-charge, comprenant les étapes consistant à :
(a) appliquer une solution de revêtement contenant des récepteurs sur des zones limitées prédéterminées du support solide,
(b) lier les récepteurs dans les revêtement limités spatialement,
(c) sécher la solution de revêtement et
(d) appliquer une solution post-charge,
un retardateur d'élution étant ajouté à la solution de revêtement, le retardateur d'élution étant choisi parmi les saccharides, la polyvinylpyrrolidone, le dextran, la gélatine ou les dérivés cellulosiques, la solution de post-charge contenant une substance de blocage.

10. Procédé selon la revendication 9, **caractérisé en ce qu'**une solution de revêtement est appliquée, contenant des récepteurs en une concentration inférieure à 90 % de la concentration limite, qui est suffisante pour couvrir complètement l'intervalle prédéterminé.

11. Phase solide, pouvant être obtenue par un procédé selon l'une des revendications précédentes, qui est produite par application d'une solution de revêtement contenant des récepteurs sur une zone prédéterminée d'un support solide non poreux et ensuite, traitement avec une solution de post-charge, les récepteurs étant disposés dans les revêtements limités spatialement, **caractérisée en ce que** ≥ 80 % des récepteurs se trouvent dans le revêtement limité spatialement.

12. Phase solide selon la revendication 11, **caractérisée en ce que** les zones limitées de façon prédéterminée contenant les récepteurs présentent un diamètre de 10 µm à 10 mm et varient de moins de 10 % d'un revêtement à l'autre.
